(19)

Europäisches
Patentamt
European
Patent Office
Office européen
des brevets

(11)  **EP 2 172 441 A1**

(12)  **EUROPEAN PATENT APPLICATION**

(43) Date of publication:
**07.04.2010 Bulletin 2010/14**

(51) Int Cl.:
**C07C 17/20** *(2006.01)*    **C07C 19/10** *(2006.01)*

(21) Application number: **08165667.0**

(22) Date of filing: **02.10.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Solvay Fluor GmbH**
**30173 Hannover (DE)**

(72) Inventors:
• **Bragante, Letanzio**
**35020 Due Carrare (PD) (IT)**

• **Peron, Sergio**
**Camponogara Venezia (IT)**
• **Ünveren, Ercan**
**30625 Hannover (DE)**

(74) Representative: **Mross, Stefan P.M. et al**
**Solvay S.A.**
**Departement Propriété Industrielle**
**310, rue de Ransbeek**
**1120 Bruxelles (BE)**

(54)  **Process for the manufacture of hydrochlorofluorocarbons using trifluoromethane as fluorinating agent**

(57)  The present invention provides a process for the manufacture of hydrochlorofluorocarbons comprising treating a hydrocarbon substituted with one or more chlorine atoms and optionally one or more fluorine atoms under elevated temperature with $GHF_3$.

EP 2 172 441 A1

**Description**

The invention relates to the fluorination of hydrochlorocarbons or hydrochlorofluorocarbons, in particular of chloroform. More specifically the invention relates to the manufacture of chlorodifluoromethane by fluorinating chloroform with trifluoromethane.

Chlorodifluoromethane ($CClF_2H$) is at the moment the most important starting material for manufacturing fluorinated monomers such as tetrafluoroethylene and perfluoropropylene, which are important monomers used for the production of fluorinated polymers and copolymers. Hence this compound is a key material in the production of many useful fluorinated polymers and copolymers. Chlorodifluoromethane is usually manufactured by fluorination of chlorinated methanes, particularly trichloromethane (chloroform) with hydrofluoric acid HF.

However, in the fluorination process of chloroform with HF a certain amount of the fully fluorinated product trifluoromethane is always formed. The amount of this by-product in the synthesis of chlorodifluoromethane depends on several factors such as the process itself (gas phase or liquid phase), the catalyst activity, temperatures and residence times of the reaction stages.

Although trifluoromethane could find some limited application as refrigerant (R23) for low temperatures itself, it is nevertheless an undesirable by-product that must be controlled and disposed in some manner. An advantageous use of trifluoromethane would thus solve many problems in the art.

Particularly, trifluoromethane is a regulated substance in the sense that its emission to the atmosphere will be strictly controlled. It has a quite long lifetime in the atmosphere and one of the highest global warming potentials (GWP). Table 1 shows the estimated atmospheric lifetimes and GWPs of the most widely used hydrofluoroalkanes versus $CO_2$ (compound HFC 23 is trifluoromethane).

| Table 1 - *From : Report of Federal Environmental Agency 20 February 2004* | | | | |
|---|---|---|---|---|
| **Compound** | **Atmospheric lifetime** | **Global** | **Warming Potential** | |
| Code | Years | 20 years | 100 years | 500 years |
| | | | | |
| $CO_2$ | | 1 | 1 | 1 |
| $CH_4$ | 12 | 56 | 21 | 6,5 |
| $N_2O$ | 120 | 280 | 310 | 170 |
| HFC 23 | 264 | 9100 | 11700 | 9800 |
| HFC 32 | 5,6 | 2100 | 650 | 200 |
| HFC 125 | 32,6 | 4600 | 2800 | 920 |
| HFC 134a | 14,6 | 3400 | 1300 | 420 |
| HFC 152a | 1,5 | 460 | 140 | 42 |
| HFC 143a | 48,3 | 5000 | 3800 | 1400 |
| HFC 227ea | 36,5 | 4300 | 2900 | 950 |
| HFC 236fa | 209 | 5100 | 6300 | 4700 |
| HFC 245ca | 6,6 | 1800 | 560 | 170 |
| HFC 365mfc | 9,9 | 2600 | 890 | 280 |
| HFC 404A | | 4760 | 3260 | 1150 |
| HFC 407C | | 3400 | 1525 | 490 |
| HFC 410A | | 3350 | 1725 | 560 |

Given the high demand in fluorinated monomers and polymers, the amount of trifluoromethane co-produced in particular in the production of chlorodifluoromethane is quite high, exceeding the small demand for it as refrigerant. Also, given the high GWP, trifluoromethane must not be released into the atmosphere but first be converted to some other less problematic compounds, which is economically unfavorable. In general, the trifluoromethane is destroyed by using high temperature, wherein carbon dioxide and hydrofluoric acid is obtained. While the resulting hydrofluoric acid can be recycled or converted to fluorides (e.g. by neutralization with lime), nevertheless the process is highly energy-consuming and significantly increases the costs of producing chlorodifluoromethane.

US 4,885,416 discloses a process for manufacturing fluorinated derivatives of alkanes. More specifically it discloses the fluorination of chloroform with HF in the presence of a catalyst, wherein one or more chlorine atoms of the chloroform are replaced by fluorine atoms. In this reaction a significant amount of trifluoromethane is obtained as a by-product, which exceeds its demand, which constitutes an economic penalty, as discussed above.

WO 2006/022763 Al also discloses a process for the manufacture of chlorodifluoromethane comprising treating chloroform with HF in the presence of a catalyst. According to WO 2006/022763 trifluoromethane is produced as a by-product in this process, which has to be separated from the chlorodifluoromethane as a vapor and removed as a waste or captured for further use. The formation of trifluoromethane is to be avoided, and therefore, in this document the process conditions and in particular the amount of catalyst are controlled to decrease the amount of trifluoromethane that is produced without substantial reduction in the chlorodifluoromethane production rate. However, even in the process of WO 2006/022763 A1 a significant amount of trifluoromethane is produced.

Thus, there is a need for an advantageous use of trifluoromethane which avoids or reduces the need to dispose of this material after its formation in the process for producing chlorodifluoromethane (or other processes). There is further a need to provide a favourable process for the production of hydrofluorocarbons and hydrofluorochlorocarbons and in particular of chlorodifluoromethane which is economically and ecologically advantageous because there is less trifluoromethane which has to be disposed.

The solution of these problems is based on the unexpected finding that the trifluoromethane that is obtained in the production of chlorodifluoromethane can be used as a fluorinating agent for fluorinating hydrocarbons substituted with one or more chlorine atoms and optionally one or more fluorine atoms and in particular of chloroform.

The trifluoromethane that is obtained as a by-product in the production of chlorodifluoromethane can thus be optionally separated and used in essentially the same process in order to increase the yield of chlorodifluoromethane by reaction with chloroform, while simultaneously decreasing the amount of trifluoromethane that must be disposed. Of course, the trifluoromethane can also be separated and used in another process for the fluorination of hydrocarbons substituted with one or more chlorine atoms and optionally one or more fluorine atoms to produce hydrochlorofluorocarbons.

The present invention thus provides a process for the manufacture of hydrochlorofluorocarbons comprising treating hydrocarbons substituted with one or more chlorine atoms and optionally one or more fluorine atoms under an elevated temperature with trifluoromethane.

The invention further provides the use of trifluoromethane as a fluorinating agent for the fluorination of hydrocarbons substituted with one or more chlorine atoms and optionally one or more fluorine atoms.

The advantages of the process of the invention are in particular :

- It implies no handling of toxic or corrosive starting material.
- It requires a starting material which is an undesired by-product of a largescale industrial process and should therefore be cheaply available.
- It does not require sophisticated equipment or special materials.

The trifluoromethane that is obtained as by-product in the production of chlorodifluoromethane can be used as fluorinating agent for nearly all hydrocarbons substituted with one or more chlorine atoms and optionally one or more fluorine atoms to obtain hydrochlorofluorocarbons. The hydrochlorofluorocarbons that can be prepared by the process of the present invention preferably are hydrochlorofluorocarbons of the general formula I

$$C_nH_mCl_oF_p \qquad (I)$$

wherein n is an integer of 1 to 5,
m is an integer of 1 to 10,
o is an integer of 1 to 10,
p is an integer of 1 to 10 and
$m+o+p=2n+2$.

In this case the process comprises treating a compound with the general formula II

$$C_nH_mCl_qF_r \qquad (II)$$

wherein n and m are defined as above,
q is an integer with $q > o$,
r is an integer (including zero) with $r < p$ and

$m + q + r = 2n + 2$

with trifluoromethane. In the above formulae I and II preferably n is an integer of 1 or 2, m is an integer of 1 to 4, o is an integer of 1 to 4, and p is an integer of 1 to 4.

With these definitions of n, m, o and p, q is thus an integer of preferably 2 to 5, and r is an integer of 0 to 4.

Most preferably, in the above formulae I and II n is 1, m is 1, o is 1 or 2 (most preferably 2), and p is 1 or 2 (most preferably 1), and q is 2 or 3 (most preferably 3), and r is 0 or 1 (most preferably 0).

As will be understood by a skilled person, the above formulae are to be interpreted that the indices n, m, o, p, r and q cannot be freely selected within the indicated ranges but only those selections are possible which meet the criteria $m + o + p = 2 n + 2$ and $m + q + r = 2 n + 2$. For a chemist this means that the above compounds are saturated and the restrictions are caused by the fact that the carbon atom has exactly four binding places.

A particularly preferred embodiment of the invention is the reaction of trifluoromethane and chloroform to chlorodifluoromethane and dichlorofluoromethane, in particular chlorodifluoromethane. The reaction scheme is as follows :

$$CF_3H + CCl_3H \rightarrow CClF_2H + CCl_2FH.$$

However, the process of the present invention is not limited to chloroform and can be adopted to other hydrochlorocarbons which are optionally substituted with fluorines, for instance chlorotrifluoroethane and dichlorotrifluoroethane. These compounds can also advantageously be fluorinated with trifluoromethane.

The temperatures used in the process of the present invention are elevated and preferably in the range of 160 to 400°C, more preferably of 180 to 360°C, more preferably of 180 to 320°C, more preferably of 180 to 280°C, more preferably of 200 to 280°C and most preferably of 220 to 260°C (such as 240°C).

The process of the present invention is preferably a continuous process.

The process is usually a gas-phase reaction carried out either in a fixed bed or in a fluidized bed reactor. These reactors are conventional reactors well known in the art. If not specified otherwise herein, the reactors are operated using their standard operating procedures known to a skilled person.

The process of the invention is preferably carried out in the presence of a catalyst. Generally, any active catalysts known in the hydrofluorination art can be employed without limitation. Preferred catalysts are fluorides of aluminium, magnesium, zinc, chromium and other metals.

The catalysts can be produced by anyone skilled in the art of catalyst-making for the fluorination process, particularly by hydrofluorination of metal precursors. The aluminium fluoride can e.g. be prepared in controlled conditions by fluorinating aluminium oxide ($Al_2O_3$) with HF. A detailed description of this preparation can be found in US 6,187,280, e.g. example 1 and the disclosure of this document is included herein by reference.

The catalysts can efficiently be supported by a suitable material such as alumina, aluminium fluoride, active carbon, chromium oxide or chromium oxyfluoride or other known catalyst support materials. The catalyst support material is preferably shaped in a useful form for the particular reactor employed.

Aluminium fluoride in particular can be both used as a catalyst and a catalyst support due to its physical appearance and properties which make it very stable in high temperature reaction environments such as hydrofluorination reactors or cracking unit reactors.

A particularly preferred embodiment of the invention is a fluorination process of chloroform using HF to produce $HCCl_2F$ and/or $HCClF_2$ and $HCF_3$ as a by-product, recovering said by-product preferably in a continuous process and treating it with chloroform to form $HCCl_2F$ and/or $HCClF_2$ increasing herewith the overall yield in the manufacture of $HCCl_2F$ and/or $HCClF_2$. With respect to the process for producing chlorodifluoromethane in which the trifluoromethane is obtained as a by-product, reference can be made to any of the known processes of the prior art, e.g. to the processes disclosed in WO 2006/022763 and US-A 4,885,416, the disclosure of which is incorporated herein by reference. Preferably, the trifluoromethane by-product is separated from the processes for producing e.g. the chlorodifluoromethane, and for this separation any of the known methods can be used. The separated trifluoromethane is then not thermally decomposed as in the prior art, but used in the process of the present invention.

Of course, the process of the present invention is not restricted to using trifluoromethane that is obtained as a by-product in the production of chlorodifluoromethane, but trifluoromethane from any source can be used. However, the most advantages are achieved in the present invention, because advantageously it is possible to use the trifluoromethane that is obtained as a by-product in an industrial process which otherwise had to be costly destroyed.

In the following the invention will be explained (for the reason of conciseness) with respect to the reaction of trifluoromethane with chloroform, however, instead of chloroform any other suitable hydrocarbon substituted with one or more chlorine atoms and optionally one or more fluorine atoms as defined above can be used.

In a preferred process of the present invention the trifluoromethane is contacted in a reactor with a fixed bed. In such a reactor the catalyst is fixed by suitable means such as a sintered disk. The ratio of trifluoromethane to chloroform is not particularly limited.

Preferably, the molar ratio of trifluoromethane to chloroform is in the range of from 0.1 to 10, more preferably in the range of from 0.5 to 10 and in particular in the range of from 0.5 to 6. Particularly preferred ranges are about 1 (about when used in this specification means ± 10 %) to about 5.

The amount of catalyst is not particularly restricted and depends e.g. on the flow rate of the chloroform and the trifluoromethane, the temperature, the type of reactor, the type of catalyst and the hydrochlorocarbon to be fluorinated. The best suitable amount of catalyst can be easily determined by a skilled person, in particular considering the examples provided below.

The reaction time depends e.g. on the type and amount of catalyst, the equipment used and the flow times. Again, the best suitable reaction time (= residence time in the reactor) can be determined by a skilled person by routine experimentation, in particular taking into account the following example.

The following example is intended to illustrate the present invention but it should not be interpreted as being restrictive.

**Example**

The reaction example has been carried out in a tubular Inconel® reactor with an internal diameter of 1/2 inch and operating in a fixed bed regime equipped internally with a sintered disk holding the catalyst in place. The reactor was kept inside an insulated tubular furnace at the desired temperature maintained by means of heating vials controlled by thermostatic electronic devices.

As the catalyst material aluminium fluoride was used. No distinct catalyst support was used in this case because of the above described properties of aluminium fluoride. The aluminium fluoride was prepared according to the method disclosed in US 6,187,280, example 1.

The amount of catalyst loaded in the trials varies from 2 up to 12 g, while the reagents flow was regulated by control devices in order to allow different values of residence times and reagents ratio.

Chloroform was fed with a micropump, and trifluoromethane was sent to the reactor measuring its rate with a flow controller. Pump and flow controller were previously calibrated, and the reagents ratio was also checked with a calibrated chromatographic system (either TCD or FID), depending on the relative amounts of the components and their separation on the analytic column.

The same equipment is used to analyze the gas product exiting the reactor.

Eventually, the gas product exiting the reactor was washed in a water absorber to determine acidity development from the reaction.

By-products and impurities of the reaction that can be formed at higher temperatures were analyzed with an MSD/GC system. For each trial table 2 shows the reaction conditions, namely temperature, residence times, catalyst amount and the selectivity and conversion obtained. The residence time was determined on the basis of the ratio of the catalyst volume to the gas flow rate at reaction conditions :

$$\text{Residence time} = \text{Catalyst volume/Volumetric flow}$$

Thus, a higher residence time means that either more catalyst was used and/or the flow rates were reduced.

| Table 2 - Reaction Results | | | | | | | | | | | | |
|---|---|---|---|---|---|---|---|---|---|---|---|---|
| IN | | | | OUT | | | | | | | | |
| | | | | Molar fractions | | | | | | Acidity | | |
| Temperature (°C) | Residence time (sec) | Catalyst amount (g) | Mole Ratio CF3H/CCl3H | CF3H | CClF2H | CCl2FH | CCl3H | others | other by GC-MSD | HCl (ppm) | HF (ppm) | η conversion fraction CF3H |
| 220 | 8,0 | 8,0 | 0,98 | 0,444 | 0,043 | 0,041 | 0,472 | | | | | 0,101 |
| 320 | 8,0 | 12,0 | 1,08 | 0,448 | 0,070 | 0,068 | 0,415 | | | | | 0,139 |
| 160 | 4,0 | 8,0 | 5,40 | 0,806 | 0,023 | 0,011 | 0,159 | | | | | 0,044 |
| 160 | 4,7 | 8,0 | 3,51 | 0,730 | 0,025 | 0,016 | 0,229 | | | | | 0,062 |
| 240 | 2,8 | 2,0 | 5,35 | 0,800 | 0,042 | 0,022 | 0,136 | | | | | 0,050 |
| 240 | 14,0 | 12,0 | 2,22 | 0,649 | 0,038 | 0,029 | 0,283 | | | | | 0,058 |
| 240 | 25,0 | 12,0 | 0,45 | 0,275 | 0,036 | 0,043 | 0,646 | | | | | 0,116 |
| 240 | 25,0 | 12,0 | 0,52 | 0,302 | 0,037 | 0,042 | 0,620 | | | | | 0,116 |
| 240 | 14,0 | 12,0 | 3,55 | 0,734 | 0,046 | 0,025 | 0,195 | | | | | 0,060 |
| 240 | 17,2 | 12,0 | 0,96 | 0,416 | 0,044 | 0,049 | 0,490 | 0,0002 | | 124 | 7,7 | 0,152 |
| 240 | 30,9 | 12,0 | 1,17 | 0,334 | 0,039 | 0,050 | 0,578 | 0,0000 | | 280 | 12,4 | 0,381 |
| 240 | 17,2 | 12,0 | 0,97 | 0,420 | 0,044 | 0,049 | 0,487 | 0,0001 | 0,0005 | 77 | 8,7 | 0,148 |
| 280 | 15,9 | 12,0 | 0,97 | 0,394 | 0,054 | 0,060 | 0,491 | 0,0007 | | 77 | 8,7 | 0,201 |
| 280 | 15,9 | 12,0 | 0,97 | 0,410 | 0,056 | 0,061 | 0,472 | 0,0009 | 0,0013 | 137 | 13 | 0,168 |
| 320 | 14,9 | 12,0 | 0,97 | 0,401 | 0,066 | 0,072 | 0,456 | 0,0045 | 0,0108 | 194 | 17 | 0,187 |
| 360 | 14,0 | 12,0 | 0,97 | 0,389 | 0,077 | 0.083 | 0,432 | 0,0194 | 0,0334 | 833 | 93 | 0,212 |

The results clearly show the useful features for the reaction and method of the present invention : Fractional conversion of trifluoromethane can be achieved and most preferably are temperatures in the range of 200 to 240°C, in particular with the catalyst used.

The conversion of reagents generally increases along with reagent trifluoromethane concentration.

Acidity is developed in the system as a function of the increased temperature and particularly low values result when operating within the temperature limit of 240°C. By-products are detected at a very low level up to a temperature limit of 280°C. Significant values of by-products emerge only at high temperatures (360°C).

The above example was carried out in a laboratory scale, however, a skilled person can easily scale up the reaction conditions to a pilot plant and an industrial scale.

**Claims**

1.  A process for the manufacture of hydrochlorofluorocarbons comprising treating a hydrocarbon substituted with one or more chlorine atoms and optionally one or more fluorine atoms under elevated temperature with $CHF_3$.

2.  The process of claim 1 wherein said hydrochlorofluorocarbon has the general formula I :

    $$C_nH_mCl_oF_p \qquad (I)$$

    wherein n is an integer of 1 to 5,
    m is an integer of 1 to 10,
    o is an integer of 1 to 10,
    p is an integer of 1 to 10 and
    $m + o + p = 2n + 2$
    and wherein the process comprises treating a hydrocarbon with the general formula II :

    $$C_nH_mCl_qF_r \qquad (II)$$

    wherein n and m are defined as above,
    q is an integer with $q > o$,
    r is an integer with $r < p$ and
    $m + q + r = 2n + 2$
    with $CHF_3$.

3.  The process of claim 1 or 2 wherein said hydrochlorofluorocarbon is $HCCl_2F$ or $HCClF_2$ or a mixture thereof and the process comprises treating chloroform with $CHF_3$.

4.  The process of any of claims 1 to 3 which is carried out at a temperature between 160°C and 400°C.

5.  The process of any of claims 1 to 4 wherein the process is a gas phase reaction carried out in a fixed bed or a fluidized bed reactor.

6.  The process of any of claims 1 to 5, wherein the hydrocarbon substituted with one or more chlorine atom and optionally one or more fluorine atoms is treated with $CHF_3$ in the presence of a catalyst.

7.  The process of claim 6, wherein the catalyst comprises a catalyst selected from the group consisting of fluorides of magnesium, aluminium, zinc, chromium and a mixture thereof.

8.  The process of claim 6 or 7 wherein the catalyst is applied on a catalyst support material selected from the group consisting of aluminium fluoride, alumina, active carbon, chromium oxide and chromium oxyfluoride.

9.  The process of any of the preceding claims, comprising treating chloroform with HF to form $HCCl_2F$ and/or $HCClF_2$ and $HCF_3$ as a by-product and using the so-formed $HCF_3$ for treating chloroform to obtain additional $HCCl_2F$ and/or $HCClF_2$.

10. The use of $CHF_3$ as a fluorinating agent in a process for the manufacture of hydrochlorofluorocarbons comprising treating a hydrocarbon substituted with one or more chlorine atoms and optionally one or more fluorine atoms under

elevated temperature with $CHF_3$.

Europäisches
Patentamt

European
Patent Office

Office européen
des brevets

**EUROPEAN SEARCH REPORT**

Application Number

EP 08 16 5667

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 3 087 976 A (MURRAY HAUPTSCHEIN ET AL) 30 April 1963 (1963-04-30) claim 1; examples; equations 1-4 ----- | 1-10 | INV. C07C17/20 C07C19/10 |
| X | US 3 466 341 A (PAUKSCH HEINRICH ET AL) 9 September 1969 (1969-09-09) column 1, lines 25-42; column 1, lines 68-column 2, line 10; example 1 ----- | 1-3,5-9 | |
| X,D | WO 2006/022763 A (DU PONT [US]; CHRISTMAS MARK JOHN [US]; DIMITRATOS YAINNIS NICOLAS [US] 2 March 2006 (2006-03-02) claim 1; page 7, lines 3-23 ----- | 1-3,5,6, 9 | |
| X | EP 0 306 566 A (DU PONT [US]) 15 March 1989 (1989-03-15) claim 1; page 3, lines 16-39; example 11 ----- | 1-9 | |
| X | DE 20 52 566 A1 (FARBWERKE HOECHST AG) 4 May 1972 (1972-05-04) example 19 ----- | 1-9 | TECHNICAL FIELDS SEARCHED (IPC) |
| X | US 3 843 546 A (SOBOLEV I ET AL) 22 October 1974 (1974-10-22) example V ----- | 1-6,9 | C07C |
| X | EP 0 129 863 A (DU PONT [US]) 2 January 1985 (1985-01-02) claim 1-4; page 6, lines 15-23; page 9, lines 10-13; example 7 ----- | 1-3,5,6, 9 | |
| X | US 3 679 767 A (VECCHIO MARTINO ET AL) 25 July 1972 (1972-07-25) claim 1; column 3, lines 23-28 ----- | 1-3,5,6 | |
| X | DE 14 93 055 A1 (SOCIEDISON) 13 March 1969 (1969-03-13) claim 1-3; page 6, lines 20-24; example 1 ----- | 1-6 | |

-/--

The present search report has been drawn up for all claims

| Place of search | Date of completion of the search | Examiner |
|---|---|---|
| Munich | 6 April 2009 | Grammenoudi, S |

EPO FORM 1503 03.82 (P04C01)

Europäisches
Patentamt

European
Patent Office

Office européen
des brevets

# EUROPEAN SEARCH REPORT

Application Number

EP 08 16 5667

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 979 271 A (HOECHST AG) 1 January 1965 (1965-01-01) claim 1,6; examples 1, 6 ----- | 1-6,9 | |
| X | EP 0 503 792 A (ICI PLC [GB]) 16 September 1992 (1992-09-16) claim 1,3,8; table 1 ----- | 1-3,5-7 | |
| X,D | US 4 885 416 A (MADER FREDERICK W [US]) 5 December 1989 (1989-12-05) claims 1,2; column 4, lines 42-44; example 1 ----- | 1-3,5,6, 9 | |
| | | | TECHNICAL FIELDS SEARCHED (IPC) |

The present search report has been drawn up for all claims

| Place of search | Date of completion of the search | Examiner |
|---|---|---|
| Munich | 6 April 2009 | Grammenoudi, S |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

..............................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 08 16 5667

This annex lists the patent family members relating to the patent documents cited in the above-mentioned European search report.
The members are as contained in the European Patent Office EDP file on
The European Patent Office is in no way liable for these particulars which are merely given for the purpose of information.

06-04-2009

| Patent document cited in search report | | Publication date | Patent family member(s) | | Publication date |
|---|---|---|---|---|---|
| US 3087976 | A | 30-04-1963 | BE | 601937 A1 | 29-09-1961 |
| | | | DE | 1468481 A1 | 19-12-1968 |
| | | | DE | 1206401 B | 09-12-1965 |
| | | | GB | 921796 A | 27-03-1963 |
| | | | NL | 142605 B | 15-07-1974 |
| | | | NL | 263002 A | |
| | | | US | 3087974 A | 30-04-1963 |
| | | | US | 3087975 A | 30-04-1963 |
| US 3466341 | A | 09-09-1969 | DE | 1618588 B1 | 10-12-1970 |
| | | | ES | 351027 A1 | 16-05-1969 |
| | | | FR | 1555412 A | 24-01-1969 |
| | | | GB | 1172215 A | 26-11-1969 |
| | | | JP | 49000164 B | 05-01-1974 |
| | | | NL | 6802023 A | 11-09-1968 |
| WO 2006022763 | A | 02-03-2006 | CN | 101010271 A | 01-08-2007 |
| | | | EP | 1868973 A1 | 26-12-2007 |
| | | | JP | 2008509995 T | 03-04-2008 |
| | | | US | 2008194779 A1 | 14-08-2008 |
| EP 0306566 | A | 15-03-1989 | JP | 1068330 A | 14-03-1989 |
| | | | US | 4678859 A | 07-07-1987 |
| DE 2052566 | A1 | 04-05-1972 | GB | 1369869 A | 09-10-1974 |
| US 3843546 | A | 22-10-1974 | NONE | | |
| EP 0129863 | A | 02-01-1985 | AU | 568365 B2 | 24-12-1987 |
| | | | AU | 2971284 A | 03-01-1985 |
| | | | BR | 8402990 A | 28-05-1985 |
| | | | CA | 1247141 A1 | 20-12-1988 |
| | | | DE | 3465184 D1 | 10-09-1987 |
| | | | ES | 8600180 A1 | 01-01-1986 |
| | | | GR | 81632 A1 | 11-12-1984 |
| | | | IN | 160224 A1 | 04-07-1987 |
| | | | JP | 2053414 B | 16-11-1990 |
| | | | JP | 60019733 A | 31-01-1985 |
| | | | MX | 163998 B | 07-07-1992 |
| | | | SU | 1577693 A3 | 07-07-1990 |
| | | | ZA | 8404715 A | 26-02-1986 |
| US 3679767 | A | 25-07-1972 | BE | 664634 A | 29-11-1965 |
| | | | CH | 490295 A | 15-05-1970 |
| | | | DE | 1493055 A1 | 13-03-1969 |
| | | | DK | 111676 B | 30-09-1968 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 08 16 5667

This annex lists the patent family members relating to the patent documents cited in the above-mentioned European search report.
The members are as contained in the European Patent Office EDP file on
The European Patent Office is in no way liable for these particulars which are merely given for the purpose of information.

06-04-2009

| Patent document cited in search report | | Publication date | Patent family member(s) | | | Publication date |
|---|---|---|---|---|---|---|
| US 3679767 | A | | FR | 1448098 | A | 16-11-1966 |
| | | | GB | 1102534 | A | 07-02-1968 |
| | | | IL | 23582 | A | 25-06-1969 |
| | | | NL | 6506321 | A | 30-11-1965 |
| | | | SE | 325868 | B | 13-07-1970 |
| DE 1493055 | A1 | 13-03-1969 | BE | 664634 | A | 29-11-1965 |
| | | | CH | 490295 | A | 15-05-1970 |
| | | | DK | 111676 | B | 30-09-1968 |
| | | | FR | 1448098 | A | 16-11-1966 |
| | | | GB | 1102534 | A | 07-02-1968 |
| | | | IL | 23582 | A | 25-06-1969 |
| | | | NL | 6506321 | A | 30-11-1965 |
| | | | SE | 325868 | B | 13-07-1970 |
| | | | US | 3679767 | A | 25-07-1972 |
| GB 979271 | A | 01-01-1965 | DE | 1256636 | B | 21-12-1967 |
| EP 0503792 | A | 16-09-1992 | CA | 2062442 | A1 | 13-09-1992 |
| | | | JP | 5178769 | A | 20-07-1993 |
| | | | US | 5254772 | A | 19-10-1993 |
| US 4885416 | A | 05-12-1989 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4885416 A
- WO 2006022763 A1
- WO 2006022763 A
- US 6187280 B